(19) Europäisches Patentamt
European Patent Office
Office européen des brevets

(11) **EP 1 881 431 A1**

(12) **EUROPEAN PATENT APPLICATION**
published in accordance with Art. 153(4) EPC

(43) Date of publication:
**23.01.2008 Bulletin 2008/04**

(51) Int Cl.:
*G06F 19/00* *(2006.01)*

(21) Application number: **05738614.6**

(22) Date of filing: **13.05.2005**

(86) International application number:
**PCT/JP2005/008785**

(87) International publication number:
**WO 2006/120752 (16.11.2006 Gazette 2006/46)**

(84) Designated Contracting States:
**AT BE BG CH CY CZ DE DK EE ES FI FR GB GR HU IE IS IT LI LT LU MC NL PL PT RO SE SI SK TR**

(71) Applicant: **Digital Information Technologies Corporation**
**Tokyo 104-0032 (JP)**

(72) Inventor: **TANAKA, Junji**
**2470071 (JP)**

(74) Representative: **Möbus, Steffen**
**Leienfelsstrasse 6**
**81243 München (DE)**

(54) **GENOME ANALYSIS SYSTEM II**

(57) [PROBLEMS] The present invention provides that the analysis can be estimated the characteristics of the originated populations which belong to the samples and the characteristics of the samples from sample data.

[MEANS FOR SOLVING PROBLEMS] By loading the sample data, by interfering with the second state variable showing the characteristics in the environment system to the first state variable showing the characteristics in the objective system, by converging the variable by selecting the maximum resonant state, and by estimating the characteristics of the samples and the characteristics of the originated populations which belong to the samples, then the result estimated the characteristics of the originated populations is outputted.

[Figure 1]

**Description**

[Field of the Invention]

The present invention relates to a genome analysis method that performs analysis for estimating the characteristics of originated populations with sample data specifically.

[Background of the Invention]

All the living organisms existing on the earth are made up of cells, and in each individual cell, there are genomes that record gene information. Cells are classified into prokaryotic cell and eukaryotic cell according to differences in their cell structure. Genomes in prokaryotic cells such as bacteria or cyanobacteria are presented in a state with no compartment in the cell; however, genomes in eukaryotic cells such as animals and plants are presented in a nucleus enclosed by a nuclear membrane.

In other word, a genome indicates one set of assembly of chromosomes thatr are essential to keep living activities. Also, the term "genome" is a compound word that is made from the words "gene" and "chromosome".

Here, the basis of life is the cell, and that cell is enclosed by a cell membrane and the nucleus is enclosed by a nuclear membrane, thus the independence of each unit is maintained. Human cells comprises differentiated and specialized cells that can be categorized into nerve cells, muscle cells, blood cells, immune system cells, epithelial cells which are cells on the surface of the skin and tissue, sensory cells, and the like according to function and form, and undifferenciated cells termed stem cells which are the source of these cells. Cell have an important aspect to change with time. That is to produce new cells through cell division. Cell division is important mechanism that make it possible to transmit and express gene information of genes.

A chromosome is inside the nucleus. That chromosome contains the gene information and genes are located on that chromosome. The genes can also be defined the method of making proteins in a genome. The basic substance that constituted a chromosome is DNA (deoxyribonucleic acid), and the genetic information is preserved in the sequences of four bases, A, T, G, and C in the DNA. A haploid living organism such as some species of bacteria and virus has one genome.

A diploid living organism has two sets of duplicating genomes. For example, a germ cell such as an egg or sperm in human has one set of genome comprising 23 types of chromosomes. A somatic cell has two sets of genomes (46 types of chromosomes). The human genome comprises approximately 3 billion DNA base pairs (3,000 mega base pairs, one mega base pairs equals one million base pairs), and the length is approximately 1 meter when arranging in one string.

A genome is the whole gene information presented in a cell, and it contains information or the like for regulating the gene and gene expression. Here, proteins and genes could be referred to as the product and the design drawing thereof, and in the genome, in addition to the design drawing, a part managing and regulating the production of the product. At the present time, the significance of that existence is unknown, however, a considerably large percentage of area that is considered to have an effect on maintaining life functions. By clarifying these, it will become possible to obtain a more accurate understanding of the life process.

Therefore, a "human genome analysis project" for analyzing the whole base sequences in human genome called human genome, and a project of "to determine the whole base sequences in all genome" are being studied for various kinds of organisms including human. Then, by unity studies between genes and proteins, it will become possible to obtain a high level of understanding of the life process.

In order for that, at first, it is considered that the network between genes should be elucidated. In other word, a plurality of proteins form the network and groups of those proteins show the specific functions. Therefore, by studying functions and corresponded information, it is possible that a gene with an unknown function may be found.

Here, the genome analysis is the overall analysis of genetic information contained in the genome of a living organism, and begins from determining the base sequences (sequences of G, A, T, and C) of DNA molecules which are constituted the genome. However, it is not easy to determine the locations and what kind of genes is from only base sequence data. Therefore, an analysis of the gene products such as messenger RNAs and proteins which are produced by transcription and translation, and a comparison of conserved base sequences across species, and furthermore an analysis based on data or the like related to individual genes that were analyzed by experimental organisms such as *escherichia coli,* a budding yeast, or the like are progressed.

Incidentally, in the case of human, approximately 3 billion pairs of DNA base sequences that are included in the total 46 chromosomes (in other words DNA molecules), which is 44 autosomal chromosome plus X chromosome and / or Y chromosome is the human genome. The genome information which we have is inherited from the genome information of the parents of one previous generation. The genome information which the parents have is inherited from the ancestors of even one previous generation. Thus, it is possible to finally reach the genome of the first living organism in 3.8 billion years ago by tracing the origin of genetic information of even previous generation in order.

As a method of performing genome analysis, Patent document 1 discloses a method of analyzing genome in which after genome sequence information is inputted, determines whether or not there is any sequence part in which a plurality (for example 10 or more bases) of identical bases are continuously arranged in the inputted the genome sequence information, and when there is a plurality of identical bases, extracts the base sequence information that comprises a predetermined number of bases that are continuously arranged at the front and back of sequence part in which the plurality of identical bases are continuously arranged, and outputs the extracted base sequence information.

By using such a genome analysis method, it is possible to detect polymorphic markers for identifying candidate genes related to diseases quickly and efficiently with an accuracy similar to SNPs (single nucleotide polymorphisms) without using SNPs.

Incidentally, the method disclosed in Patent document 1 is a method of analyzing genome that detects polymorphic markers for identifying candidate genes related to diseases; however, in the analysis, it is sometimes necessary to analyze approximately 3 billion base pairs of the DNA base sequences from various viewpoints. Therefore, since it is predicted that there are many methods to perform various genome analyses which are still undiscovered, the discovery of those method is anticipated.

Patent document 1: Japanese Patent Application No. 2003-288346

[Description of the Invention]

[Problems to be solved by the Invention]

Taking into consideration the above situation, the object of the present invention is to provide a genome analysis system and analysis method that is capable of estimating the characteristics of originated populations and the characteristics of samples from sample data.

[Means for Solving the Problem]

Solving the aforementioned problems, the present invention had following compositions.

The gist of invention described in claim 1 is that a genome analysis system, comprising: a load means of loading the sample data; and an estimation means of estimating the characteristics of the samples and the characteristics of the originated populations by selecting the first state variable showing the characteristics of the samples which constitute the originated populations and the second state variable showing the characteristics of the originated populations and by interfering with the second state variable to the first state variable and converging the first state variable.

The gist of invention described in claim 2 is that the genome analysis system according to claim 1, wherein an estimation means of estimating the characteristics of the samples and the characteristics of the originated populations to select (observe) the maximum resonant state of the first state variable by interfering with the second state variable to the first state variable.

The gist of invention described in claim 3 is that the genome analysis system according to claim 1 or 2, wherein the first state variable is the diplotype frequency of each sample and the second state variable is the haplotype frequency in the originated populations.

The gist of invention described in claim 4 is that the genome analysis system according to claim 1 to 3, further comprising a polymorphisms determination means of determining the gene polymorphisms to examine; an allele information determination means of determining by a wet process of the gene polymorphisms in the populations desired to examine; a parameter determination means of determining the characteristics parameter of the samples and the characteristics parameter of the originated populations; an interference means of interfering with the characteristic parameter of the originated populations to the characteristic parameter of the samples; a selection means of selecting the maximum resonant state of the characteristic parameter of the samples; an analysis means of calculating the characteristic parameter of the samples; and an estimation means of estimating the characteristic of the samples and the characteristic of the originated populations by calculating the characteristic parameter of the originated populations from the calculated characteristic parameter of the samples.

The gist of invention described in claim 5 is that the genome analysis system according to claim 1 to 4, further comprising an interference means of diagonalizing the sample matrix by calculating a direct product by multiplying the originated populations matrix showing the haplotype frequency in the originated populations which belong to the samples to the sample matrix showing the diplotype frequency of each sample; and an estimation means of specifying the diplotype frequency by using the maximum component in the diagonal component of the diagonalized matrix as the diplotype frequency to calculate.

The gist of invention described in claim 6 is that a genome analysis method, comprising: a load step of loading the sample data; and an estimation step of estimating the characteristics of the samples and the characteristics of the originated populations by selecting the first state variable showing the characteristics of the samples which constitute the originated populations and the second state variable showing the characteristics of the originated populations and by interfering with the second state variable to the first state variable and converging the first state variable.

The gist of invention described in claim 7 is that the genome analysis method according to claim 6, wherein a selection step of selecting (observing) the maximum resonant state of the first state variable by interfering with the second state variable to the first state variable.

The gist of invention described in claim 8 is that the genome analysis method according to claim 6 or 7, wherein the first state variable is the diplotype frequency of each sample and the second state variable is the haplotype frequency in the originated populations.

The gist of invention described in claim 9 is that the genome analysis method according to claim 6 to 8, further comprising a polymorphisms determination step of determining the gene polymorphisms to examine; an allele information determination step of determining by a wet process of the gene polymorphisms in the populations desired to examine; a parameter determination step of determining the characteristics parameter of the samples and the characteristics parameter of the originated populations; an interference step of interfering with the characteristic parameter of the originated populations to the characteristic parameter of the samples; a selection step of selecting the maximum resonant state of the characteristic parameter of the samples; a result analysis step of calculating the characteristic parameter of the samples; and estimating the characteristic of the samples and the characteristic of the originated populations by calculating the characteristic parameter of the originated populations from the calculated characteristic parameter of the samples.

The gist of invention described in claim 10 is that the genome analysis method according to claim 6 to 9, further comprising a step of specifying the diplotype frequency by diagonalizing the sample matrix by calculating a direct product by multiplying the originated populations matrix showing the haplotype frequency of the originated populations which belong to the samples to the sample matrix showing the diplotype frequency of each sample and by using the maximum component in the diagonal component of the diagonalized matrix as the diplotype frequency to calculate.

The gist of invention described in claim 11 is that a storage medium storing an computable program of the genome analysis method according to claim 6 to 10.

In the genome analysis method II of the present invention, since the characteristics of originated populations and the characteristics of samples can be estimated by interfering the first state variable showing the characteristics of samples with the second state variable showing the characteristics of originated populations, that is by calculating a direct product, a difficult calculation process is only once. Therefore, it is possible to examine for the characteristics of originated populations and the characteristics of samples at extremely higher speed than conventional methods.

Moreover, while the number of diplotype samples which can be determined at once is limited to about 20 in the conventional methods, the greater number of diplotype samples can be calculated at once in the method of the present invention.

[Description of the preferred embodiments]

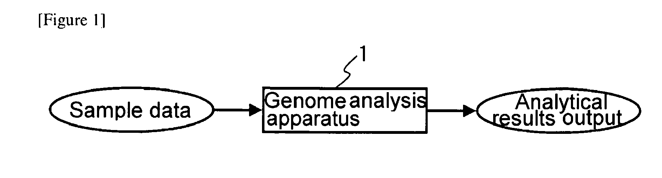

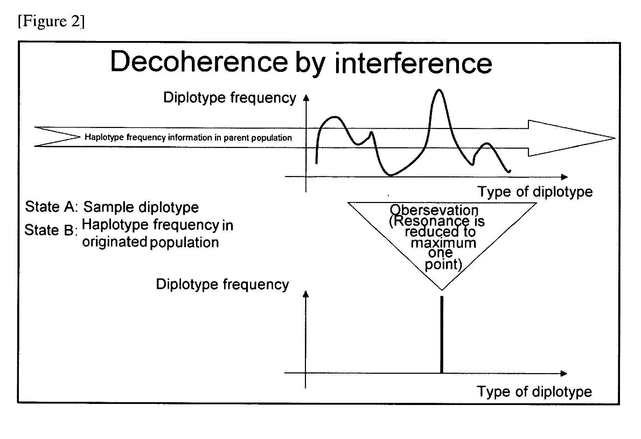

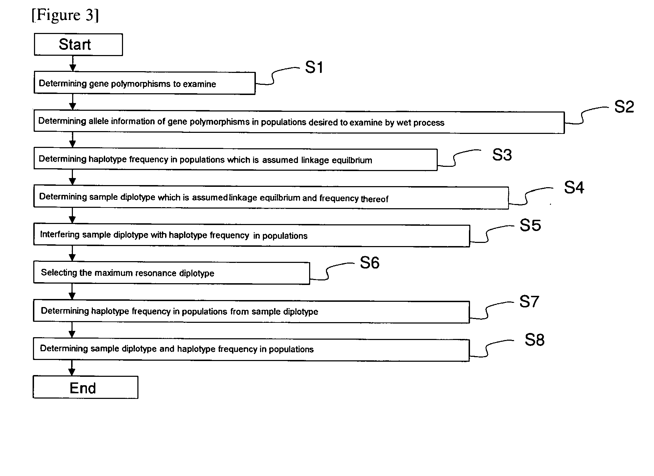

Embodiments of the present invention are described herein. Figure 1 illustrates an outline of the genome analysis apparatus used for the genome analysis method in the present invention, Figure 2 illustrates an outline of the analysis by the genome analysis apparatus shown in Figure 1, and Figure 3 illustrates flow charts showing the genome analysis method in the present invention.

As shown in Figure 1, a genome analysis apparatus 1 estimates the characteristics of originated populations which belong to sample data and the characteristic of sample data from sample data, and then outputs the analytical result thereof. As a genome analysis apparatus 1, a notebook PC, a desktop PC, or the like equipped an analysis program to perform operations for the below-mentioned genome analysis can be used.

For example, as shown in Figure 2, an outline of the analysis by the genome analysis apparatus 1 shows a model of an observation status which can be characterized by convergence to the maximum resonant state by the interference state, and the characteristics of samples and the characteristics of originated populations are estimated by interfering with the state A which is an objective system to the state B which is an environmental system and by converging to the maximum resonant state between state A and state B.

Here, a state variable A is a diplotype in each sample, and a state variable B is a haplotype frequency in originated populations. Then, the maximum resonant state is selected by interfering with the state A to the state B, which the details are described below.

Therefore, originated populations which belong the sample data is supposed to be a system which can be modeled in Hilbert space. A haplotype H of the populations demonstrates as a vector whose component is a haplotype frequency. The type of haplotype is set as $h_m$, the haplotype frequency thereof aligned in the originated populations is represented as a vector, and as shown below in equation 1, the transversely aligned object is represented as bra vector and the longitudinally aligned object is represented as ket vector.

[Equation 1]

$$\begin{pmatrix} h_1 & h_2 & h_3 & - & h_{m'} \end{pmatrix} \rightarrow \langle h_{m'} | \quad : \text{Bra vector}$$

$$\begin{pmatrix} h_1 \\ h_2 \\ | \\ h_m \end{pmatrix} \rightarrow | h_m \rangle \quad : \text{Ket vector}$$

Here, a projection operator to a haplotype vector is represented as a product of ket vector and bra vector as shown in equation 2, and an actual value is a matrix of $H_{mm'}$.

[Equation 2]

**Projection operator to haplotype vector**

**Product of ket vector and bra vetcor** $| h_m \rangle \langle h_m |$ **is actually matrix of** $(H_{mm'})$.

Then, the sample diplotype and the frequency thereof is represented as a direct product in a vector space whose basis is a haplotype type ($h_l$), that is, a matrix of a direct product of $h_l$ and $h_{l'}$. Thus, the sample diplotype frequency is represented as a matrix of $D_{ll'}$ as shown in equation 3. Each component $D_{ll'}$ of this matrix is a frequency with a diplotyle consisted of a pair of haplotype h, and haplotype $h_{l'}$.

[Equation 3]

**Sample diplotype and the frequency**

**Expressed as direct product in vector space** $(h_l \otimes h_{l'})$ **whose basis is haplotype type** $(h_l)$.

**Thus, sample diplotype frequency is expressed as matrix** $(D_{ll'})$.

Then, when an operator of $D_{ll'}$ is represented as D and an operator of $H_{mm'}$ is represented as H, a mapping of D to H in HD, that is, H component in D is calculated. However, an operation between matrices becomes a matrix which does not have only a diagonal component and cannot be calculated an eigenvalue. Therefore, the theory of quantum mechanics observation or the convergence principle of an interference and a wave packet is applied. That is, D is the data which should be observed in an objective system and H is in an environmental system or an interference factor. The eigenvalue (resonant value) of D is calculated by interfering and operating with H to D and then by diagonalizing (decoherence) of D and having only a diagonal component. Moreover, the extraction of data actually observed from the eigenvalue (a convergence of wave packet) is observed as the maximum resonant state which is based on the maximum resonance. That is, the interference is exactly calculated the direct product of $D_{ll'}$ and $H_{mm'}$ as shown in equation 4. Furthermore, the convergence means performing this diagonalization. This is a matrix showing actually observed system which is the direct product of equation 4 represented the system showing the state of the constructional system of the objective system D and an interference system (an environmental system) H in greater detail.

[Equation 4]

$$\left| D_{ll'} \right\rangle \otimes \left| H_{mm'} \right\rangle$$

To select the eigenvalue of the maximum resonance from a plurality of eigenvalues of D herein is due to changing to the matrix showing the actually observed value of matrix D revealed an objective form only when the observation is conducted in a quantum system. That is, the matrix showing the state in an objective system means not changing only by diagonalization with interference.

In view of interference system that is generally macroscopic and the component is not overlapping, the interference system observed each D component is considered to be relatively prime and independent. That is, as shown in equation 5, it can be considered to be represented the direct product of each D component and the corresponding H component. This means that a haplotype $h_{il}$ or $h_{il'}$ which is a diplotype component of individual (i) is interfered (effected) only by $H_{ll}$ or $H_{ll}$ $H_{l'l''}$ which is an equivalent component of D.

[Equation 5]

$$\left| D_{11} \right\rangle \otimes \left| H_{11} \right\rangle \;+\; \left| D_{12} \right\rangle \otimes \left| H_{12} \right\rangle \;+\; - \; - \; - \; -$$

Then, the genome analysis method by the genome analysis apparatus 1 is explained. First, as shown in Figure 3, the gene polymorphisms to examine is determined (Step S1). Here, an allele information of the gene polymorphisms in a group desired to examine is determined by a wet process (Step S2). Moreover, the haplotype frequency in the populations assumed a linkage equilibrium is determined from the allele information (Step S3).

Subsequently, the diplotype and the frequency thereof of the samples assumed a linkage equilibrium from the allele information is determined (Step S4). Moreover, the haplotype frequency in the populations is made to interfere with the sample diplotype (Step S5).

Here, the maximum resonant diplotype is selected (Step S6). Then, the haplotype frequency in the populations is determined from the sample diplotype (Step S7). Then, the sample feature parameter and the sample parameter in the populations is calculated (Step S8).

[Embodiment 1]

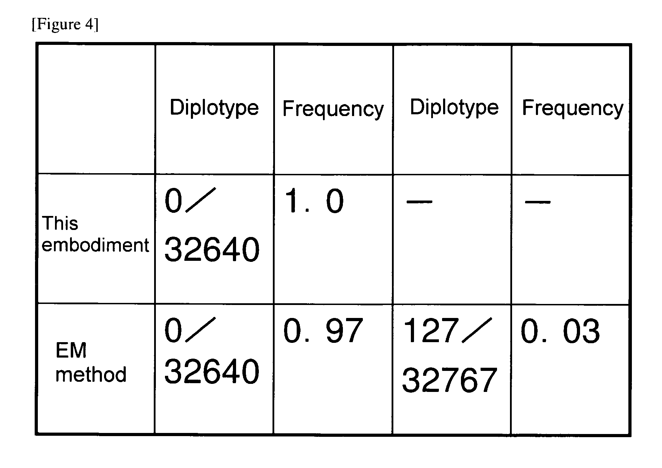

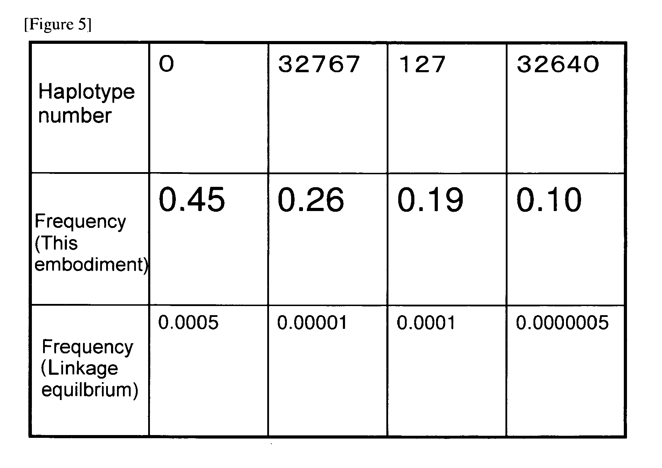

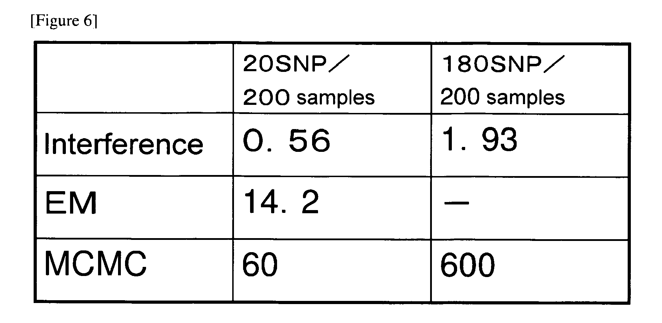

Then, an embodiment is explained. Figures 4-6 as shown below are a drawing showing an example of analytical results by the genome analysis method to estimate the sample diplotype, to use the genotype data and allele information of a plurality of loci for calculating the haplotype frequency in the originated populations, and to converge the maximum resonance state by interfering.

In the gene analysis, a major purpose is to perform mapping the genotype data (for example, disease gene) to the phenotype data (presence of disease). In this case, although the haplotype which is a perfect data should be used as a genotype data, but then a single gene data is generally obtained in an economical aspect and a technical aspect.

Therefore, it is necessary to estimate the haplotype from the relationship of single gene polymorphism data as a part of the analysis. When the haplotype was estimated, while the MCMC method based on Bayes statistics, the EM method based on the maximum likelihood method, or the like is used, the maximum resonance choice method by interference was used in this embodiment.

In this case, the vector expression on Hilbert space was used as showing the haplotype frequency in the populations and the sample diplotype. Moreover, the projection operation of the haplotype frequency in the populations was made to interfere with the sample diplotype, and used as diagonalization of sample diplotype matrix. This is because an inherent state (frequency) is assumed to be resonated by interference.

Here, the method of estimating the state of the maximum resonance as a sample diplotype under the assumption that the actual existence of the state with an inherent state of the strongest resonance. This is because the matrix showing the state is not change when the state value is not determined.

In this embodiment, as mentioned above, the populations haplotype frequency $h_m$ and the sample diplotype $d_{ll'}$ are used as two state which is resonated by interference.

Here, as mentioned above, $h_m$ and $d_{ll'}$ are considered that one state characterized in the objective system and one state characterized in the environment system. In that manner, $h_m$ and $d_{ll'}$ can be considered to convert to a matrix in an inherent state that an objective system should be observed by an environment system as a quantum system.

Then, $h_m$ and $d_{ll'}$ is assumed by the formula (1) and the formula (2) in equation 6, and is estimated from the linkage equilibrium state.

[Equation 6]

$$\begin{pmatrix} h_1 \\ h_2 \\ | \\ h_m \end{pmatrix} \rightarrow |h_m\rangle \quad \bullet \bullet \bullet \quad (1)$$

$$\left(D_{ll'}\right) \rightarrow (h_1 \otimes h_{1'}) \quad \bullet \bullet \bullet \quad (2)$$

Moreover, the direct product in equation 4 is considered that an inherent state in the objective system and the corresponding environmental state is selected by interfering. It is considered that the inherent state of the maximum value (resonance) is selected from the inherent state in this separated objective system.

[Equation 7]

$$|D_{11}\rangle \otimes |H_{11}\rangle + |D_{12}\rangle \otimes |H_{12}\rangle + - - - + |D_{mm}\rangle \otimes |H_{mm}\rangle$$

Then, by using these formulas, at Step 1, a linkage equilibrium is assumed and $h_m$ is calculated as an initial value from the allele information on each gene polymorphism. At Step 2, $d_{ll'}$ assumed a linkage equilibrium is calculated as an initial value from $h_m$.

Then, the initial value $h_m$ is interfered with the initial value $d_{ll}$, $d_{ll'}$ is objectivize, then $d_{ll'}$ of the maximum resonance value is selected and determined, and then hm is performed the statistical processing and determined.

Then, the estimated data of haplotype frequency is explained below.

Figure 4 shows the example of the diplotype and the frequency thereof in the estimated samples. While several types of haplotype and the possibility frequency thereof are estimated by the conventional EM method or the like, the sample diplotype is estimated in only one in this embodiment.

Figure 5 shows that the example of the haplotype in the originated populations in this embodiment and the estimated frequency thereof, and the frequency of the haplotype in case of the linkage equilibrium.

Figure 6 shows that the computing time of the same data in this embodiment, the EM method, and the MCMC method. In case of using SNPs as gene polymorphisms, this embodiment demonstrates the convergence of computing time comparing to the other methods in 20 loci. In 180 loci, the EM method cannot be substantively performed and this embodiment demonstrates the greatest shortening of computing time comparing to the MCMC method.

Thus, in this implementation, by loading sample data, interfering with the first state variable in the objective system by the second state variable in the environment system, selecting the maximum resonance, and then converging the state variable of samples in the objective system, since it was made to output the result estimated the second state variable showing the characteristic of the originated populations in the environmental system, the analysis for estimating the characteristics of samples and the characteristics of the originated populations which belong to the samples can be performed by the sample data.

[Industrial applicability]

According to the present invention, the analysis for estimating the characteristic of the originated populations which belong to the samples and the characteristic of samples can be performed by the sample data described above.

[Brief Description of the Drawings]

[Figure 1]
It is a drawing illustrating the outline of a genome analysis apparatus used for the genome analysis method in the present invention.
[Figure 2]
It is a drawing illustrating the outline of the analysis by the genome analysis apparatus of Figure 1.
[Figure 3]
It is a flow chart illustrating the genome analysis method in the present invention.
[Figure 4]
It is a drawing illustrating the diplotype and frequency of the samples in the present invention.
[Figure 5]
It is a drawing illustrating the haplotype and frequency in the originated populations which belong to the samples in the present invention.
[Figure 6]
It is a drawing illustrating the computing time in the present invention.

[Description of Notations]

1    Genome analysis apparatus

**Claims**

1. A genome analysis system, comprising: a load means of loading the sample data; and an estimation means of estimating the characteristics of the samples and the characteristics of the originated populations by selecting the first state variable showing the characteristics of the samples which constitute the originated populations and the second state variable showing the characteristics of the originated populations and by interfering with the second state variable to the first state variable and converging the first state variable.

2. The genome analysis system according to claim 1, wherein an estimation means of estimating the characteristics of the samples and the characteristics of the originated populations to select (observe) the maximum resonant state of the first state variable by interfering with the second state variable to the first state variable.

3. The genome analysis system according to claim 1 or 2, wherein the first state variable is the diplotype frequency of each sample and the second state variable is the haplotype frequency in the originated populations.

**4.** The genome analysis system according to claim 1 to 3, further comprising a polymorphism determination means of determining the gene polymorphisms to examine; an allele information determination means of determining by a wet process of the gene polymorphisms in the populations desired to examine; a parameter determination means of determining the characteristics parameter of the samples and the characteristics parameter of the originated populations; an interference means of interfering with the characteristic parameter of the originated populations to the characteristic parameter of the samples; a selection means of selecting the maximum resonant state of the characteristic parameter of the samples; an analysis means of calculating the characteristic parameter of the samples; and an estimation means of estimating the characteristic of the samples and the characteristic of the originated populations by calculating the characteristic parameter of the originated populations from the calculated characteristic parameter of the samples.

**5.** The genome analysis system according to claim 1 to 4, further comprising an interference means of diagonalizing the sample matrix by calculating a direct product by multiplying the originated populations matrix showing the haplotype frequency in the originated populations which belong to the samples to the sample matrix showing the diplotype frequency of each sample; and an estimation means of specifying the diplotype frequency by using the maximum component in the diagonal component of the diagonalized matrix as the diplotype frequency to calculate.

**6.** A genome analysis method, comprising: a load step of loading the sample data; and an estimation step of estimating the characteristics of the samples and the characteristics of the originated populations by selecting the first state variable showing the characteristics of the samples which constitute the originated populations and the second state variable showing the characteristics of the originated populations and by interfering with the second state variable to the first state variable and converging the first state variable.

**7.** The genome analysis method according to claim 6, wherein a selection step of selecting (observing) the maximum resonant state of the first state variable by interfering with the second state variable to the first state variable.

**8.** The genome analysis method according to claim 6 or 7, wherein the first state variable is the diplotype frequency of each sample and the second state variable is the haplotype frequency in the originated populations.

**9.** The genome analysis method according to claim 6 to 8, further comprising a polymorphisms determination step of determining the gene polymorphisms to examine; an allele information determination step of determining by a wet process of the gene polymorphisms in the populations desired to examine; a parameter determination step of determining the characteristics parameter of the samples and the characteristics parameter of the originated populations; an interference step of interfering with the characteristic parameter of the originated populations to the characteristic parameter of the samples; a selection step of selecting the maximum resonant state of the characteristic parameter of the samples; a result analysis step of calculating the characteristic parameter of the samples; and estimating the characteristic of the samples and the characteristic of the originated populations by calculating the characteristic parameter of the originated populations from the calculated characteristic parameter of the samples.

**10.** The genome analysis method according to claim 6 to 9, further comprising a step of specifying the diplotype frequency by diagonalizing the sample matrix by calculating a direct product by multiplying the originated populations matrix showing the haplotype frequency in the originated populations which belong to the samples to the sample matrix showing the diplotype frequency of each sample and by using the maximum component in the diagonal component of the diagonalized matrix as the diplotype frequency to calculate.

**11.** A storage medium storing a computable program of the genome analysis method according to claim 6 to 10.

[Figure 1]

[Figure 2]

# Decoherence by interference

Diplotype frequency

Haplotype frequency information in parent population

Type of diplotype

State A: Sample diplotype
State B: Haplotype frequency in
originated population

Obersevation
(Resonance is
reduced to
maximum
one
point)

Diplotype frequency

Type of diplotype

[Figure 3]

```
            ┌──────────────┐
            │    Start     │
            └──────────────┘
                   │
                   ▼
┌────────────────────────────────────────────────┐
│ Determining gene polymorphisms to examine      │ ⟍ S1
└────────────────────────────────────────────────┘
                   │
                   ▼
┌────────────────────────────────────────────────────────────────────────────────┐
│ Determining allele information of gene polymorphisms in populations desired to   │ ⟍ S2
│ examine by wet process                                                           │
└────────────────────────────────────────────────────────────────────────────────┘
                   │
                   ▼
┌─────────────────────────────────────────────────────────────────┐
│ Determining haplotype frequency in populations which is assumed  │ ⟍ S3
│ linkage equilbrium                                               │
└─────────────────────────────────────────────────────────────────┘
                   │
                   ▼
┌──────────────────────────────────────────────────────────────────────┐
│ Determining sample diplotype which is assumed linkage equilbrium and   │ ⟍ S4
│ frequency thereof                                                      │
└──────────────────────────────────────────────────────────────────────┘
                   │
                   ▼
┌───────────────────────────────────────────────────────────┐
│ Interfering sample diplotype with haplotype frequency in   │ ⟍ S5
│ populations                                                │
└───────────────────────────────────────────────────────────┘
                   │
                   ▼
┌────────────────────────────────────────────┐
│ Selecting the maximum resonance diplotype   │ ⟍ S6
└────────────────────────────────────────────┘
                   │
                   ▼
┌───────────────────────────────────────────────────────────┐
│ Determining haplotype frequency in populations from sample │ ⟍ S7
│ diplotype                                                  │
└───────────────────────────────────────────────────────────┘
                   │
                   ▼
┌───────────────────────────────────────────────────────────┐
│ Determining sample diplotype and haplotype frequency in    │ ⟍ S8
│ populations                                                │
└───────────────────────────────────────────────────────────┘
                   │
                   ▼
            ┌──────────────┐
            │     End      │
            └──────────────┘
```

[Figure 4]

|  | Diplotype | Frequency | Diplotype | Frequency |
|---|---|---|---|---|
| This embodiment | 0／32640 | 1. 0 | — | — |
| EM method | 0／32640 | 0. 97 | 127／32767 | 0. 03 |

[Figure 5]

| Haplotype number | O | 32767 | 127 | 32640 |
|---|---|---|---|---|
| Frequency (This embodiment) | 0.45 | 0.26 | 0.19 | 0.10 |
| Frequency (Linkage equilbrium) | 0.0005 | 0.00001 | 0.0001 | 0.0000005 |

[Figure 6]

|  | 20SNP／<br>200 samples | 180SNP／<br>200 samples |
|---|---|---|
| Interference | 0. 56 | 1. 93 |
| EM | 14. 2 | — |
| MCMC | 60 | 600 |

## INTERNATIONAL SEARCH REPORT

| International application No. |
| --- |
| PCT/JP2005/008785 |

A. CLASSIFICATION OF SUBJECT MATTER
Int.Cl⁷ G06F19/00

According to International Patent Classification (IPC) or to both national classification and IPC

B. FIELDS SEARCHED

Minimum documentation searched (classification system followed by classification symbols)
Int.Cl⁷ G06F19/00

Documentation searched other than minimum documentation to the extent that such documents are included in the fields searched
Jitsuyo Shinan Koho        1922-1996   Jitsuyo Shinan Toroku Koho   1996-2005
Kokai Jitsuyo Shinan Koho  1971-2005   Toroku Jitsuyo Shinan Koho   1994-2005

Electronic data base consulted during the international search (name of data base and, where practicable, search terms used)
PubMed, JSTPlus(JOIS)

C. DOCUMENTS CONSIDERED TO BE RELEVANT

| Category* | Citation of document, with indication, where appropriate, of the relevant passages | Relevant to claim No. |
| --- | --- | --- |
| A | ITO, T. et al., Association Test Algorithm Between a Qualitative Phenotype and a Haplotype or Haplotype Set Using Simultaneous Estimation of Haplotype Frequencies, Diplotype Configura tions and Diplotype-Based Penetrances, Genetics, Vol.168, 2004.12, pages 2339 to 2348 | 1-11 |

☐ Further documents are listed in the continuation of Box C.  ☐ See patent family annex.

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 27 May, 2005 (27.05.05) | 14 June, 2005 (14.06.05) |

| Name and mailing address of the ISA/ | Authorized officer |
| --- | --- |
| Japanese Patent Office | |
| Facsimile No. | Telephone No. |

Form PCT/ISA/210 (second sheet) (January 2004)

**EP 1 881 431 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003288346 A